# FASCICULE DE BREVET EUROPEEN

(11) **EP 0 669 126 B1**
(45) Date de publication et mention de la délivrance du brevet: **27.01.1999**
(21) Numéro de dépôt: 95400061.8
(22) Date de dépôt: 12.01.1995
(51) Int. Cl.: A61K 7/48, A61K 7/06

(54) **Emulsion stabilisée, destinée à hydrater la peau et son utilisation**
Stabilisierte Emulsion zur Hauthydratation
Stabilized emulsion for hydrating the skin

(30) Priorité: 31.01.1994 FR 9401030
(43) Date de publication de la demande: 30.08.1995
(73) Titulaire: L'OREAL, 75008 Paris (FR)
(72) Inventeur: Nadaud, Jean-François, F-92140 Clamart (FR); Laugier, Jean-Pierre, F-92160 Antony (FR); Le Royer, Isabelle, F-78350 Jouy-en-Josas (FR); Bernard, Dominique, F-75015 Paris (FR)
(74) Mandataire: Lhoste, Catherine

(56) Documents cités:
- WO-A-93/19730
- US-A- 4 370 319
- US-A- 5 254 331
- S.T.N., Serveur de Bases de Données, Karlsruhe, DE, Fichier Chemical Abstracts, vol 119 n 146379 & JP-A-05139949 (KAO CORP) * résumé *
- DATABASE WPI Week 9244, Derwent Publications Ltd., London, GB; AN 92-360690 & JP-A-04 261 111 (KANEBO)
- S.T.N., Serveur de Bases de Données, Karlsruhe, DE, Fichier Chemical Abstracts, vol 115, n 141986 & JP-A-03063208 (KOBAYASHI KOSE) * résumé *

## Description

La présente invention se rapporte à une émulsion cosmétiquement et/ou dermatologiquement acceptable, stabilisée et destinée à hydrater la peau du visage et/ou du corps humain, voire même du cuir chevelu, et des ongles.

Plus précisément, elle contient une phase grasse, une phase aqueuse, au moins un émulsionnant et au moins un hydratant.

L'invention se rapporte aussi à une utilisation cosmétique de cette émulsion pour lutter contre le vieillissement, l'acné, la pigmentation de la peau et/ou la chute des cheveux et à un procédé pour hydrater la peau.

On sait combien il est important pour la peau d'être bien hydratée à la fois en superficie et dans les couches plus profondes de l'épiderme et du derme. Aussi, il est nécessaire de lui apporter des actifs hydratants.

Au cours du vieillissement de la peau, il apparaît différents signes sur la peau, très caractéristiques de ce vieillissement, se traduisant notamment par une modification de la structure et de la fonction cutanée. Ce vieillissement est de nature physiologique mais il est également photoinduit, c'est-à-dire dû à l'exposition répétée de la peau à la lumière et, par conséquent, à la formation de radicaux libres oxygénés par action de cette lumière sur les constituants de la peau.

Les principaux signes cliniques de vieillissement cutané sont notamment les suivants : apparition de rides profondes en augmentation avec l'âge. On constate en particulier une désorganisation du "grain" de la peau, c'est-à-dire que le micro-relief est moins régulier et présente un caractère anisotrope.

Par ailleurs, le teint de la peau est généralement modifié, il apparaît plus pâle et plus jaune, ce qui semble être dû essentiellement à une désorganisation de la microcirculation (moins d'hémoglobine au niveau du derme papillaire). De nombreuses taches colorées apparaissent en surface, ce qui est dû à une mélanogénèse altérée. Il existe sur certaines zones des irritations diffuses et parfois des telangiectasies.

Un autre signe clinique de vieillissement est l'aspect sec et rêche de la peau qui est dû essentiellement à une desquamation plus importante, ces squames en diffractant les rayons lumineux participent aussi à l'aspect un peu gris du teint.

Par ailleurs, on cherche de plus en plus souvent à introduire dans des compositions cosmétiques et/ou dermatologiques des actifs comme des vitamines telles que les vitamines A, B, C, D, E ou F pour apporter des traitements spécifiques contre le vieillissement de la peau, son déssèchement, contre l'acné et certaines maladies de la peau (psoriasis), ou pour favoriser la cicatrisation des plaies et/ou la restructuration de la peau.

En particulier, la présence d'acide ascorbique ou vitamine C, notamment sur la peau, en quantité suffisante permet de stimuler la croissance du tissu conjonctif, et notamment celle du collagène.

L'acide ascorbique permet également de renforcer les défenses du tissu cutané contre les agressions extérieures telles que celles des rayonnements ultra-violet, de la pollution ainsi que les agressions des médicaments, de l'alcool ou du tabac.

Or un certain nombre de réactifs hydratants tels que la vitamine C sont connus pour leur instabilité.

Ainsi, l'article "Stability of ascorbic acid" de BR. HAJRATWALA paru dans "Sciences Pharmaceutiques Revue" pages 281-286, enseigne que l'acide ascorbique possède des propriétés d'instabilité en milieu aqueux, aérobie ou anaérobie, avec une instabilité plus prononcée en milieu aérobie.

Il y est illustré en particulier le comportement de l'acide ascorbique face à des variations du pH de la solution le contenant, des variations de lumière, de température, face à des composés tels que des tensio-actifs, des solvants, des catalyseurs notamment métalliques.

Un tel comportement de l'acide ascorbique en milieu aqueux, entraîne la nécessité de le stabiliser. Ceci a déjà été proposé dans les brevets JP 89/115 558 et JP 83/129 892 qui enseignent, d'une part le blocage du site réactif de l'acide ascorbique, à savoir le site hydroxyle, par estérification et/ou éthérification avec notamment des dérivés phosphatés, sulfatés ou alkylés et, d'autre part l'emploi de ces dérivés dans des compositions cosmétiques pour jouer le même rôle que celui de la vitamine C. Ce blocage du site actif permet ainsi de rendre l'acide ascorbique plus stable ; mais moins efficace que la vitamine C à l'état libre (c'est-à-dire sans groupements additionnels).

D'autres types de stabilisation de l'acide ascorbique ont déjà été proposés, et notamment dans le document "Bioconversion of a vitamin to vitamins C and E in skin" de KAKUJI TOJO et AE-RIC. LEE publié dans J. Cosmet. Chem., 38, pages 333 - 339, comme l'estérification d'un dérivé d'acide ascorbique, et d'un dérivé de tocophérol avec l'acide phosphorique.

Mais l'utilisation de ce type de diester dans des compositions cosmétiques ne permet pas la libération rapide et en quantité suffisante d'acide ascorbique à la surface de la peau.

Par ailleurs, l'acide ascorbique et le rétinol sont connus pour provoquer des irritations de la peau lorsqu'ils sont utilisés dans des compositions cosmétiques et/ou dermatologiques, à l'état libre et à une concentration élevée.

D'autres actifs comme les nucléotides sont difficiles à solubiliser. D'autres encore comme certaines vitamines sont difficilement biodisponibles.

Par ailleurs, on connaît du document JP-A-05-139949 une composition cosmétique blanchissante contenant une huile de silicone, de la glycérine et de l'ascorbylphosphate de magnésium. Cette composition ne permet pas une bonne hydratation de la peau.

Par conséquent, il subsiste le besoin d'une émulsion hydratante cosmétique et/ou dermatologique qui permet la libération, avec un bon rendement, à partir d'un précurseur, d'une quantité importante d'actif par hydrolyse enzymatique, au contact du *Stratum corneum.*

La demanderesse a découvert de façon surprenante que l'utilisation dans la phase aqueuse d'une émulsion, d'un composé phosphaté apte à libérer par réaction enzymatique au contact de la peau, un actif autre que l'agent hydratant favorisant l'hydratation de la peau, et dans la phase grasse de l'émulsion, d'au moins une gomme siliconée et/ou une huile perfluorée, permettait d'hydrater la peau de façon efficace tout en assurant également un bon rendement de l'hydrolyse enzymatique du précurseur de l'actif.

La présence dans l'émulsion selon l'invention d'une gomme siliconée et/ou d'huile perfluorée permet d'améliorer le rendement de l'hydrolyse enzymatique lorsque l'émulsion est appliquée sur la peau, et donc d'augmenter son rôle d'hydratant.

La phosphatase acide cutanée possède un bon rendement dans la cinétique d'hydrolyse de précurseurs d'actifs phosphatés, tels que l'ascorbyl phosphate de magnésium, dans un milieu ayant une température d'environ 30°C et un taux d'humidité relative d'environ 90 %.

Aussi, pour être dans les conditions de température et d'humidité optimum du *Stratum corneum* et proches de celles nécessaires à un bon rendement de l'hydrolyse enzymatique, il est nécessaire d'associer à l'actif phosphaté des émulsionnants ou tensioactifs et des hydratants particuliers.

L'émulsion selon l'invention donne de bons résultats, quelque soit le type de peau sur laquelle elle est appliquée.

Selon l'invention on peut choisir un ou plusieurs composés phosphatés, choisis parmi les phosphates de vitamine, les nucléotides, les hydroxyacétones phosphatées, les glycérophosphates (béta-glycérol phosphate disodique tétrahydrate) et leurs mélanges.

Parmi les phosphates de vitamines, on peut utiliser les phosphates d'acide ascorbique, les phosphates de tocophérol, les phosphates de rétinol, les phosphates de vitamine D, les phosphates de vitamine B, les phosphates de vitamine F. Parmi les nucléotides, on peut utiliser l'adénosine phosphate, la guanosine phosphate, la cytosine phosphate, l'uridine phosphate, la thymidine phosphate, l'inosine phosphate, la xanthosine phosphate.

Préférentiellement, le composé phosphaté utilisé dans l'émulsion selon l'invention est un phosphate d'acide ascorbique, et plus précisément l'ascorbyl phosphate d'un métal alcalin ou alcalino-terreux comme le magnésium, le potassium, le sodium, le lithium.

L'huile perfluorée entrant dans la phase grasse est par exemple un perfluoropolyéther tel que le perfluoro-polyméthylisopropyl éther, ou un perfluoroalcane.

La gomme siliconée ayant un poids moléculaire important est par exemple une diméthiconol (ou polydiméthylsiloxane-ol), telle que la gomme de silicone vendue sous la dénomination commerciale QC F2-1671 par Dow Corning).

L'émulsion selon l'invention peut également contenir une huile siliconée. Celle-ci peut être choisie parmi les huiles siliconées volatiles ou non, cycliques ou non, telles que les penta- ou hexa-diméthylsiloxane, ou les polydiméthylsiloxanes de viscosité d'au plus 0,06m²/s.

L'émulsion selon l'invention peut également contenir une huile végétale choisie parmi les fractions liquides de beurre de karité, l'huile d'abricot, l'huile de pépins de cassis, l'huile de jojoba, l'huile de sésame, l'huile de macadamia, l'huile de tournesol, l'huile de rosier muscat.

Les émulsionnants utilisables dans l'invention sont de préférence des tensioactifs non-ioniques, préservant l'activité enzymatique mise en jeu.

Ces tensioactifs non-ioniques sont en particulier choisis parmi des tensioactifs oxyéthylénés de formule générale :

R₁ - (O - CH₂ - R₂)ₙ - OH

où n est un entier allant de 1 à 100.
R₁ est choisi parmi les radicaux alkyles linéaires ou ramifiés ayant de 8 à 30 atomes de carbone, les radicaux alcènes linéaires ou ramifiés ayant de 8 à 30 atomes de carbone, un atome d'hydrogène, un groupement aryle éventuellement substitué par un radical alkyle R₃ ayant de 1 à 12 atomes de carbone, un goupement hétérocyclique, un groupement 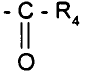 où R₄ est un groupement alkyle ayant de 8 à 22 atomes de carbone ;
R₂ est choisi parmi les radicaux alkyles linéaires ou ramifiés ayant de 1 à 30 atomes de carbone, les radicaux alcènes linéaires ou ramifiés ayant de 1 à 30 atomes de carbone, éventuellement hydroxylés.

De préférence, R₁ est choisi parmi l'hydrogène, un radical alkyle en C₁₂, un groupement phényle substitué par un radical alkyle en C₈ et le groupement 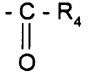 où R₄ est un akyle en C₁₁ à C₁₇.

De préférence, R₂ est choisi parmi :
CH_{2 ;}

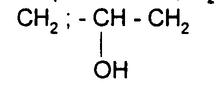

De même, de façon avantageuse n vaut : 1, 9, 23 ou 100.

Les tensioactifs oxyéthylénés, utilisés selon l'invention, ont de 1 à 30 moles d'oxyde d'éthylène et de préférence 8 à 12 moles d'oxyde d'éthylène.

Plus spécialement, ces tensioactifs oxyéthylénés sont obtenus par réaction entre un alcool polyhydrique (glycérol, sorbitane oxyéthyléné, polyéthylène glycol) et un acide gras en C₁₂ à C₂₂ (l'acide stéarique, l'acide oléique, l'acide laurique), ou parmi les alcools oxyéthylénés (l'alcool laurique à 23 moles d'oxyde d'éthylène, l'octyl phényle à 9 moles d'oxyde d'éthylène, le dodécanediolpolyglycèrolé à 3,5 moles d'oxyde d'éthylène, le polyéthylèneglycol à 6 moles d'oxyde d'éthylène).

Les émulsionnants peuvent également être choisis parmi les esters d'ose.

Parmi les esters d'ose, on peut utiliser notamment les esters d'ose en C₃ à C₆, comportant éventuellement une chaîne carbonée en C₁ à C₃₀. Ces esters d'ose peuvent être choisis, par exemple, parmi le dodécylmaltoside, le dioléate de méthylglucose, le monooléate de sorbitane polyoxyéthyléné à 20 moles d'oxyde d'éthylène.

Le ou les agents hydratants sont utilisés dans l'émulsion selon l'invention de façon à préserver l'activité de la phosphatase acide cutanée et en améliorer son rendement. En particulier, il est choisi parmi les agents humectants, classiquement utilisés dans les domaines cosmétique et/ou dermatologique.

Parmi les agents hydratants, on peut par exemple utiliser le propylène glycol, les alcools polyhydriques polyoxyéthylénés, la vaseline, les huiles minérales, le pyroglutamate de sodium, l'acide hyaluronique, les dérivés de chitosane, le lactamide, I'acétamide, la thiamorpholinone, les acides carboxyliques alpha hydroxylés ayant de 3 à 20 atomes de carbone, les polyols (glycérine, sorbitol, D-panthénol, diglycérine, mannitol, inositol, aminopolyol), les polyosides, les protéines telles que le collagène, les polyolosides tels que les alginates, les lipoprotides.

L'émulsion selon l'invention peut également contenir des adjuvants cosmétiquement et/ou dermatologiquement acceptables choisis parmi des gélifiants et épaississants (polymères carboxyvinyliques, poly(méth)acrylates de glycéryle, polyacrylamides) autres qu'une gomme de silicone, des parfums, des charges (talc), des conservateurs, des électrolytes simples (NaCl, NaOH, KCl), les co-émulsionnants.

Pour améliorer la stabilisation de l'émulsion, on peut en outre ajouter un ou plusieurs stabilisants tels qu'un mélange de dioxyde de titane et de poudre d'amidon.

L'émulsion selon l'invention peut se présenter sous forme d'une émulsion huile dans eau (H/E) ou eau dans huile (E/H) contenant éventuellement des sphérules (liposomes, nanocapsules, nanosphères) dans lesquelles les agents hydratants et/ou d'autres actifs peuvent être introduits.

La proportion de la phase grasse est comprise dans une gamme allant de 5 % à 25 % en poids, et de préférence de 6 % à 12 % en poids par rapport au poids total de l'émulsion.

L'agent hydratant est choisi en pratique dans une gamme allant de 0,1 % à 20 % en poids, et de préférence de 1 % à 10 % en poids par rapport au poids total de l'émulsion.

L'émulsionnant est choisi en particulier dans une gamme allant de 0,1 % à 10 % en poids, et de préférence de 0,1 % à 3 % en poids par rapport au poids total de la composition.

Le composé phosphaté est choisi de préférence dans une gamme allant de 0,01% à 10 % en poids, et de préférence de 0,01 % à 1 % en poids par rapport au poids total de la composition.

Les adjuvants sont choisis séparément dans une gamme allant de 0,05 % à 2 % en poids, et de préférence choisis dans une gamme allant de 0,05 % à 0,5 % en poids par rapport au poids total de l'émulsion.

De préférence, la phase huileuse de l'émulsion contient 1/6 d'huile perfluorée, 1/6 d'huile végétale et 2/3 d'huile de silicone.

L'émulsion selon l'invention se rapporte également à une composition cosmétique et/ou dermatologique pour lutter contre le vieillissement, I'acné, la pigmentation et/ou la chute des cheveux et à une utilisation de l'émulsion définie ci-dessus pour lutter contre ces conséquences de la déshydratation.

Cette invention se rapporte aussi à un procédé pour hydrater la peau, et qui consiste à appliquer sur la peau l'émulsion selon l'invention.

L'invention va maintenant être décrite de façon plus détaillée, au moyen d'exemples. Les quantités y sont donnés en pourcentage pondéral.

### Exemple 1 : Emulsion E/H

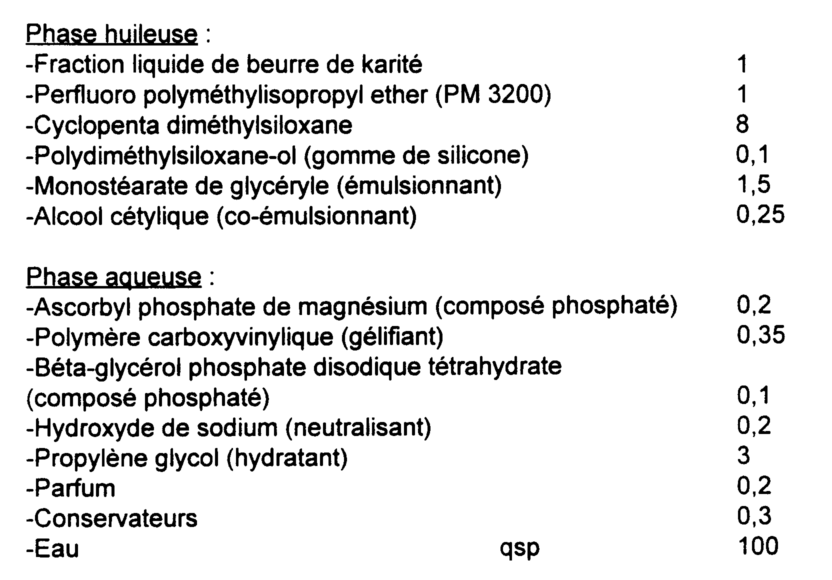

Cette émulsion se présente sous forme d'une crème blanche, destinée à l'hydratation de la peau du visage.

### Exemple 2 : Emulsion H/E

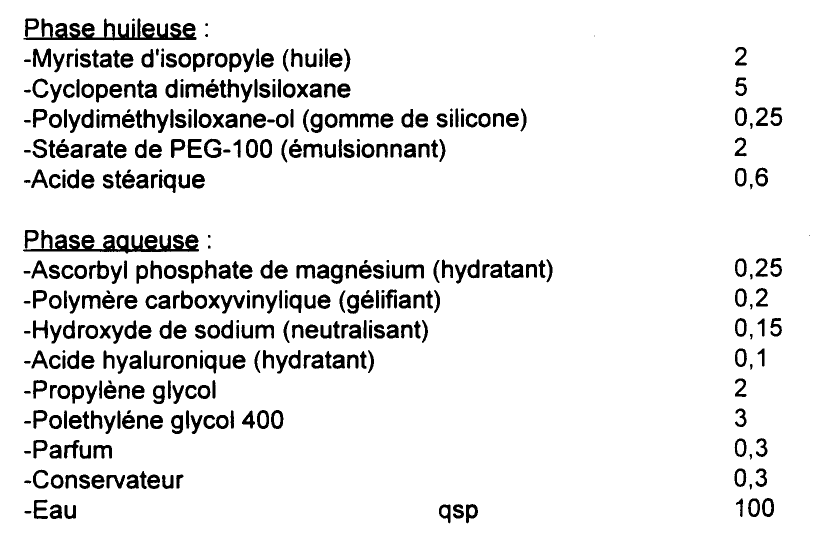

Cette émulsion se présente sous forme d'une crème blanche destinée à l'hydratation de la peau du corps humain.

### Exemple 3 : Emulsion H/E

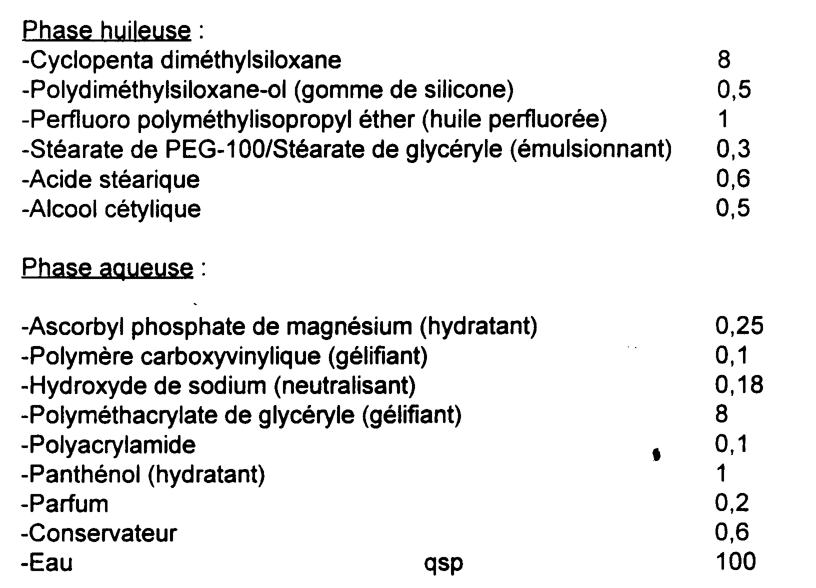

Cette émulsion se présente sous forme d'une crème blanche destinée à l'hydratation du visage.

Un test d'hydratation a été réalisé avec la crème de l'exemple 3 (crème A) comparativement à une crème ayant une composition identique mais sans gomme de silicone ni huile perfluorée (crème B).

Ce test a consisté à mesurer, au Dermodiag (mesure de l'impédance) et à l'Infraanalyzer 450 (mesure du pic de l'eau par infra-rouge), l'hydratation de la peau après application des crèmes sur la face interne de l'avant-bras des 15 sujets du panel.

Dans un premier temps, les mesures ont été faites 5 h (Infraanalyzer) et 24 h (Dermodiag) après traitement. On a déterminé le pourcentage d'augmentation de l'hydratation. Les résultats sont les suivants :

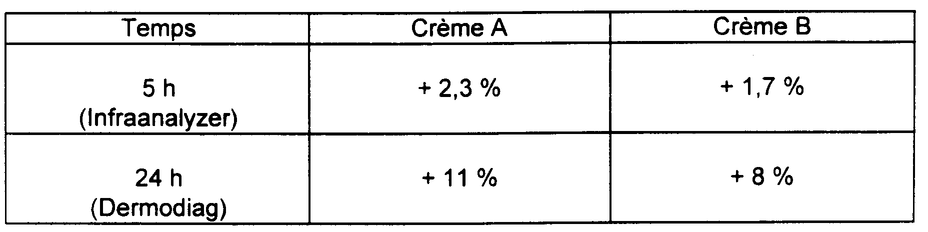

Ces résultats montrent que les deux crèmes sont hydratantes, mais que l'hydratation se prolonge davantage avec la crème selon l'invention. La comparaison fait donc apparaître une supériorité d'hydratation signifcative avec la crème selon l'invention.

Un test à long terme a également été réalisé et il a consisté à mesurer, au Dermodiag (mesure de l'impédance), l'hydratation de la peau après application des crèmes sur la face interne de l'avant-bras des 15 sujets du panel pendant 18 jours consécutifs.

Les mesures ont été réalisées au 18ième jour, c'est-à-dire à l'arrêt du traitement, et au 24ième jour, c'est-à-dire trois jours après l'arrêt du traitement.

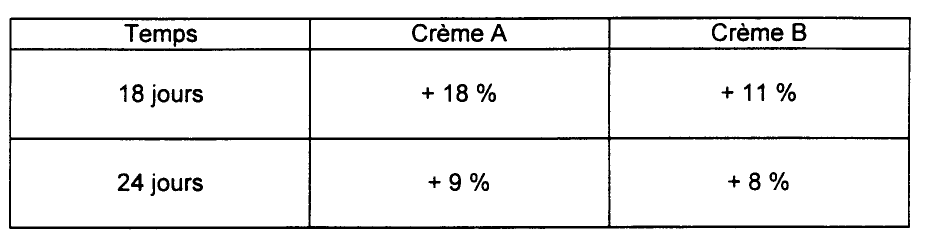

La comparaison entre les deux produits fait apparaître une supériorité de la crème selon l'invention après 18 jours de traitement.

## Revendications

1. Emulsion hydratante cosmétiquement et/ou dermatologiquement acceptable, stabilisée contenant une phase grasse, une phase aqueuse, au moins un émulsionnant, au moins un agent hydratant, caractérisée en ce que la phase aqueuse contient au moins un composé phosphaté apte à libérer, par réaction enzymatique au contact de la peau, un actif différent de l'agent hydratant, favorisant l'hydratation de la peau, ce composé étant choisi parmi les phosphates de vitamine, les nucléotides, les hydroxyacétones phosphatées, les glycérophosphates et leurs mélanges, et en ce que la phase grasse contient en outre au moins un constituant choisi parmi les huiles perfluorées, les gommes siliconées, et leurs associations.

2. Emulsions selon la revendication 1, caractérisée en ce que le composé phosphaté est un phosphate d'acide ascorbique.

3. Emulsion selon l'une des revendications 1 et 2, caractérisée en ce que l'émulsion contient à la fois une huile perfluorée et une gomme siliconée.

4. Emulsion selon l'une des revendications 1 à 3, caractérisée en ce que l'émulsion contient en outre une huile siliconée.

5. Emulsion selon l'une des revendications 1 à 4, caractérisée en ce qu'elle contient, de plus, une huile végétale.

6. Emulsion selon l'une des revendications 1 à 5, caractérisée en ce que l'émulsionnant est choisi parmi les tensio-actifs non ioniques oxyéthylénés et les esters d'ose.

7. Emulsion selon l'une des revendications 1 à 6, caractérisée en ce que l'émulsionnant est un alcool gras oxyéthyléné ayant de 1 à 30 moles d'oxyde d'éthylène.

8. Emulsion selon l'une des revendications 1 à 7, caractérisée en ce que l'agent hydratant est un polyol.

9. Emulsion selon l'une des revendications 1 à 8, caractérisée en ce que l'agent hydratant est la glycérine.

10. Emulsion selon l'une des revendications 1 à 9, caractérisée en ce que l'hydratant est choisi dans la gamme allant de 0,1 % à 20 % en poids par rapport au poids total de l'émulsion.

11. Emulsion selon l'une des revendications 1 à 10, caractérisée en ce que le composé phosphaté est choisi dans une gamme allant de 0,01 % à 10 % en poids par rapport au poids total de l'émulsion.

12. Emulsion selon l'une des revendications 1 à 11, caractérisée en ce que l'émulsionnant est choisi dans une gamme allant de 0,1 % à 10 % en poids par rapport au poids total de l'émulsion.

13. Emulsion selon l'une des revendications 1 à 12, caractérisée en ce qu'elle contient un adjuvant choisi parmi les conservateurs, les parfums, les charges, les gélifiants et épaississants autre qu'une gomme de silicone, les électrolytes simples, les co-émulsionnants.

14. Emulsion selon l'une des revendications 1 à 13, caractérisée en ce qu'elle contient un hydratant, un composé phosphaté, une huile perfluorée, une gomme de silicone, un tensioactif non-ionique oxyéthyléné.

15. Utilisation de l'émulsion selon l'une des revendications 1 à 14, pour lutter contre le vieillissement, l'acné, la pigmentation de la peau et/ou la chute des cheveux, le dessèchement de la peau.

16. Procédé pour hydrater la peau qui consiste à appliquer sur la peau l'émulsion selon l'une des revendications 1 à 14.

17. Composition cosmétique et/ou dermatologique pour lutter contre le vieillissement, l'acné, la pigmentation de la peau et/ou la chute des cheveux, caractérisée en ce qu'elle consiste en une émulsion selon l'une quelconque des revendications 1 à 14.

## Claims

1. Stabilized, cosmetically and/or dermatologically acceptable moisturizing emulsion containing a fatty phase, an aqueous phase, at least one emulsifying agent and at least one moisturizing agent, characterized in that the aqueous phase contains at least one phosphated compound capable of releasing, via an enzymatic reaction when it comes into contact with the skin, an active agent other than the moisturizing agent, which promotes moisturization of the skin, this compound being chosen from vitamin phosphates, nucleotides, phosphated hydroxyacetones, glycerophosphates and mixtures thereof, and in that the fatty phase also contains at least one constituent chosen from perfluoro oils and silicone-containing gums, and combinations thereof.

2. Emulsions according to Claim 1, characterized in that the phosphated compound is an ascorbic acid phosphate.

3. Emulsion according to either of Claims 1 and 2, characterized in that the emulsion contains both a perfluoro oil and a silicone-containing gum.

4. Emulsion according to one of Claims 1 to 3, characterized in that the emulsion also contains a silicone-containing oil.

5. Emulsion according to one of Claims 1 to 4, characterized in that it additionally contains a plant oil.

6. Emulsion according to one of Claims 1 to 5, characterized in that the emulsifying agent is chosen from oxyethylenated nonionic surfactants and saccharide esters.

7. Emulsion according to one of Claims 1 to 6, characterized in that the emulsifying agent is an oxyethylenated fatty alcohol containing from 1 to 30 mol of ethylene oxide.

8. Emulsion according to one of Claims 1 to 7, characterized in that the moisturizing agent is a polyol.

9. Emulsion according to one of Claims 1 to 8, characterized in that the moisturizing agent is glycerol.

10. Emulsion according to one of Claims 1 to 9, characterized in that the moisturizing agent is chosen within the range of from 1% to 20% by weight relative to the total weight of the emulsion.

11. Emulsion according to one of Claims 1 to 10, characterized in that the phosphated compound is chosen within a range of from 0.01% to 10% by weight relative to the total weight of the emulsion.

12. Emulsion according to one of Claims 1 to 11, characterized in that the emulsifying agent is chosen within a range of from 0.1% to 10% by weight relative to the total weight of the emulsion.

13. Emulsion according to one of Claims 1 to 12, characterized in that it contains an adjuvant chosen from preserving agents, fragrances, fillers, gelling and thickening agents other than a silicone gum, simple electrolytes and co-emulsifying agents.

14. Emulsion according to one of Claims 1 to 13, characterized in that it contains a moisturizing agent, a phosphated compound, a perfluoro oil, a silicone gum and an oxyethylenated nonionic surfactant.

15. Use of the emulsion according to one of Claims 1 to 14 to combat ageing of the skin, acne, pigmentation of the skin and/or hair loss, and drying of the skin.

16. Process for moisturizing the skin which consists in applying to the skin the emulsion according to one of Claims 1 to 14.

17. Cosmetic and/or dermatological composition for combating ageing of the skin, acne, pigmentation of the skin and/or hair loss, characterized in that it consists of an emulsion according to any one of Claims 1 to 14.

## Patentansprüche

1. Kosmetisch und/oder dermatologisch akzeptable, stabilisierte, hydratisierende Emulsion, die eine Fettphase, eine wäßrige Phase, mindestens einen Emulgator und mindesten ein Hydratisierungsmittel enthält, dadurch gekennzeichnet, daß die wäßrige Phase mindestens eine Phosphatverbindung enthält, die befähigt ist, im Kontakt mit der Haut durch eine enzymatische Reaktion einen Wirkstoff freizusetzen, der von dem Hydratisierungsmittel verschieden ist und die Hydratisierung der Haut begünstigt, wobei diese Verbindung unter den Phosphaten von Vitaminen, Nucleotiden, phoshathaltigen Hydroxyacetonen und Glycerophosphaten und deren Gemischen ausgewählt ist, und dadurch, daß die Fettphase ferner mindestens einen Bestandteil enthält, der unter den perfluorierten Ölen, den Silicongummis und deren Gemischen ausgewählt ist.

2. Emulsion nach Anspruch 1, dadurch gekennzeichnet, daß die Phosphatverbindung ein Ascorbinsäurephosphat ist.

3. Emulsion nach einem der Ansprüche 1 und 2, dadurch gekennzeichnet, daß die Emulsion gleichzeitig ein perfluoriertes Öl und einen Silicongummi enthält.

4. Emulsion nach einem der Ansprüche 1 bis 3, dadurch gekennzeichnet, daß die Emulsion ferner ein Siliconöl enthält.

5. Emulsion nach einem der Ansprüche 1 bis 4, dadurch gekennzeichnet, daß sie ferner ein pflanzliches Öl enthält.

6. Emulsion nach einem der Ansprüche 1 bis 5, dadurch gekennzeichnet, daß der Emulgator unter den ethoxylierten, nichtionischen grenzflächenaktiven Stoffen und den Zuckerestern ausgewählt ist.

7. Emulsion nach einem der Ansprüche 1 bis 6, dadurch gekennzeichnet, daß der Emulgator ein ethoxylierter Fettalkohol mit 1 bis 30 mol Ethylenoxid ist.

8. Emulsion nach einem der Ansprüche 1 bis 7, dadurch gekennzeichnet, daß das Hydratisierungsmittel ein Polyol ist.

9. Emulsion nach einem der Ansprüche 1 bis 8, dadurch gekennzeichnet, daß das Hydratisierungsmittel Glycerin ist.

10. Emulsion nach einem der Ansprüche 1 bis 91 dadurch gekennzeichnet, daß das Hydratisierungsmittel in einer Menge im Bereich von 0,1 bis 20 Gew.-%, bezogen auf das Gesamtgewicht der Emulsion, ausgewählt wird.

11. Emulsion nach einem der Ansprüche 1 bis 10, dadurch gekennzeichnet, daß die Phosphatverbindung in einer Menge im Bereich von 0,01 bis 10 Gew.-%, bezogen auf das Gesamtgewicht der Emulsion, ausgewählt wird.

12. Emulsion nach einem der Ansprüche 1 bis 11, dadurch gekennzeichnet, daß der Emulgator in einer Menge im Bereich von 0,1 bis 10 Gew.-%, bezogen auf das Gesamtgewicht der Emulsion, ausgewählt wird.

13. Emulsion nach einem der Ansprüche 1 bis 12, dadurch gekennzeichnet, daß sie einen Zusatzstoff enthält, der unter den Konservierungsmitteln, Füllstoffen, Gelbildnern und Verdickungsmitteln, die von den Silicongummis verschieden sind, einfachen Elektrolyten und Coemulgatoren ausgewählt ist.

14. Emulsion nach einem der Ansprüche 1 bis 13, dadurch gekennzeichnet, daß sie ein Hydratisierungsmittel, eine Phosphatverbindung, ein perfluoriertes Öl, einen Silicongummi und einen nichtionischen, ethoxylierten grenzflächenaktiven Stoff enthält.

15. Verwendung der Emulsion nach einem der Ansprüche 1 bis 14 zur Bekämpfung von Hautalterung, Akne, Pigmentierung der Haut und/oder von Haarausfall und dem Austrocknen der Haut.

16. Verfahren zur Hydratisierung der Haut, das darin besteht, auf die Haut die Emulsion nach einem der Ansprüche 1 bis 14 aufzutragen.

17. Kosmetische und/oder dermatologische Zusammensetzung zur Bekämpfung von Hautalterung, Akne, Pigmentierung der Haut und/oder Haarausfall, dadurch gekennzeichnet, daß sie aus einer Emulsion nach einem der Ansprüche 1 bis 14 besteht.
